Europäisches Patentamt

European Patent Office

Office européen des brevets

(11) Publication number: **0 487 935 A1**

## EUROPEAN PATENT APPLICATION

(21) Application number: **91118715.1**

(22) Date of filing: **04.11.91**

(51) Int. Cl.5: **C07C 273/04**, B01J 4/00, B01J 10/00

(30) Priority: **26.11.90 CH 3726/90**

(43) Date of publication of application: **03.06.92 Bulletin 92/23**

(84) Designated Contracting States: **AT DE ES FR GB IT NL**

(71) Applicant: **UREA CASALE S.A.** Via Della Posta 4

CH-6900 Lugano(CH)

(72) Inventor: **Pagani, Giorgio** Salita dei Frati 13 **CH-6900 Lugano(CH)**

(74) Representative: **Incollingo, Italo** Piazzale Lavater, 3 **I-20129 Milano(IT)**

(54) Process and high-yield reactors for the synthesis of urea.

(57) A process is described (and related reactors) for the high-yield synthesis of urea from ammonia and carbon dioxide fed pure to a "once through" reactor, i.e. without any recycling, operating at high pressure and temperature, characterized by the fact that the pure ammonia is fed to the reaction space by being first circulated substantially over all the internal wall or reactor shell, the temperature of said ammonia and its circulation being such as to: a) cool said reactor shell in such a way that it no longer requires costly material lining; and b) not to affect appreciably the reaction.

FIG. 2

This invention concerns a process and the related high-yield reactor for the production of urea through the synthesis of ammonia (NH3) and carbon dioxide (CO2) said reactor comprising substantially an internally lined shell resistant to high pressure and high temperature conditions.

More particularly the invention concerns a reactor for the synthesis of urea of the "once through" type, fed only by pure reagents NH3 and CO2, operating at pressures above 250 bar and temperatures above 160°C.

In a recent Patent application by the Applicant under the title "New process for the production of urea with variable yields and its applications even in existing plants" a new process for the production of urea is described, characterized by the fact that part of the urea production is achieved in a high-yield "once through" reactor without recycling, i.e. fed only with pure reagents.

In order better to fix ideas, once through reactors have been largely used in the past and many of these are still in operation; reference can be made, for example, to the so-called "Vulcan" reactor, which, as is well known, operates at 400 kg/cm2 and 215°C with a molar ratio NH3/CO2 = 4.5 and with 80% yields. Given the high operating temperature (about 25°C above that of conventional recycling reactors) the reactor is lined internally with zirconium, so as to avoid the corrosion phenomena which would occur by using less costly material (more particularly Sandvik 2RE69 steel, urea grade AISI 316, etc.), even when using the appropriate means to passivate the walls (for example by using passivating oxygen O2 with or without H2O2).

By reasons of heat balance, the reactor must be fed with relatively cold reagents (CO2 at 120÷150°C and liquid NH3 at ≈ 80°C).

The high-yield reactor described above offers maximum reliability from the point of view of corrosion thanks to its zirconium lining. However, owing to the high cost of zirconium, the total cost of the equipment proves very high, to the point of affecting considerably the total investment with references to the new varied yield process.

Surprisingly, it has now been found that it is possible to maintain the same reliability of the zirconium lining by using less costly materials (for example urea grade AISI 316, 2RE69, etc.) and conventional passivation systems (O2) by lowering by at least 15÷20°C the operating temperature of the metal in contact with the process by using an additional lining (jacket) forming an airspace in which to circulate the relatively cool ammonia.

In a preferred embodiment, the reactor is provided with an additional protective lining in ordinary material (stainless steel) (jacket) which, instead of being rigidly welded to the inner wall of the pressure shell, now consists of an evelope (jacket) welded to said shell, lined conventionally and with ordinary material (for example AISI 316, 2RE69 steel, etc.) in the upper part and free to dilate in the lower part so as to create a gap between the shell and the jacket.

According to an aspect of the invention, feed NH3 is made to flow in said gap or airspace at a relatively low temperature, for example below 100°C, better if around 80°C, thus cooling the additional protective lining and the envelope or reactor shell sufficiently to avoid corrosion phenomena (for example at 20÷30°C lower than the operating temperature).

According to another aspect of the invention the heat developed by the synthesis reaction and exchanged by the NH3 is kept at relatively low values so as not to affect the reactor's operating temperature. Advantageously such exchanged heat will be found in any case in the ammonia being reacted and will permit a better equalization of the temperature along the reaction zones.

The conventional distribution plates may be maintained in thin zirconium, since they operate at maximum temperature (215÷220°C).

The various aspects and advantages of the invention will appear more clearly from the description of the embodiments (preferred and not limitative) shown in the drawings accompanying this Application, these being two schematic views partially in cross-section with a projection containing the reactor's longitudinal axis.

In the illustrations M indicates the conventional pressure resistant shell in ordinary steel material. Inside M is found an envelope or jacket (CAM) in stainless steel not particularly costly (AISI 316, 2RE69, etc.) characterized by the fact that:

- only the upper part (SA) of the jacket (CAM) is welded in annular fashion to the cylindrical end zone (not bell-shaped) of M and is closed;

- the lower end (EI) of (CAM) remains substantially open and may dilate freely towards the bottom with respect to the shell;

- between CAM's outer wall (open at the bottom and closed at the top) and M's inner wall is created the airspace or gap (GAP).

According to an aspect of the invention, in a zone 10 slightly under the welding (SA) the liquid synthesis ammonia NH3 is introduced at a relatively low temperature (for example at about 80°C) which by circulating in the GAP and leaving from EI at the bottom cools the entire cylindrical wall of M.

According to another aspect of the invention, the temperature and circulation of NH3 are such that not only they cool M to the point where it no longer requires a costly zirconium lining but only a conventional steel one, but moreover they don't affect adversely the reaction temperature of the synthesis reagents. Advantageously only the upper dome-shaped part (CU) unaffected by the circulation of liquid NH3 must be zirconium-lined; this high-quality lining (RZ) covers however a decidedly minor part of the urea reactor (RU). The conventional plates (PZ) are maintained in zirconium but their thickness is never very considerable. The CO2 is fed from the bottom of RU through duct 1 and distributor (D).

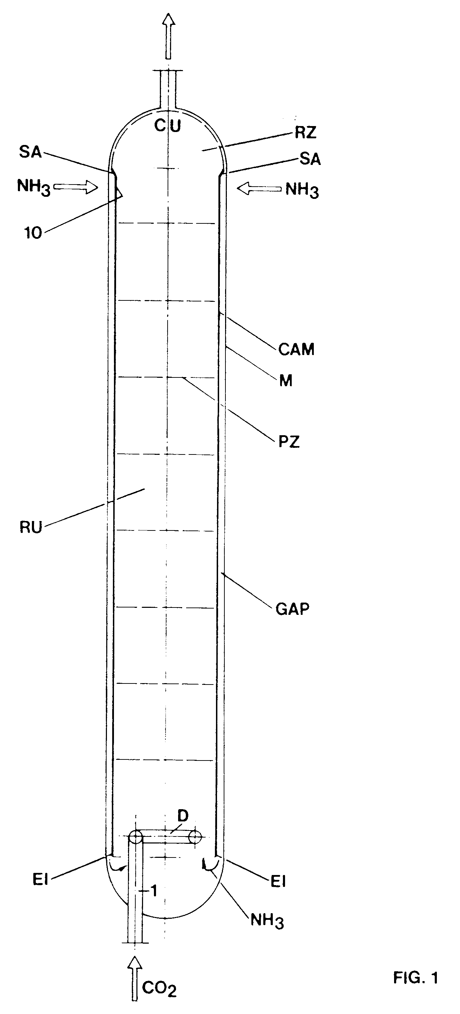

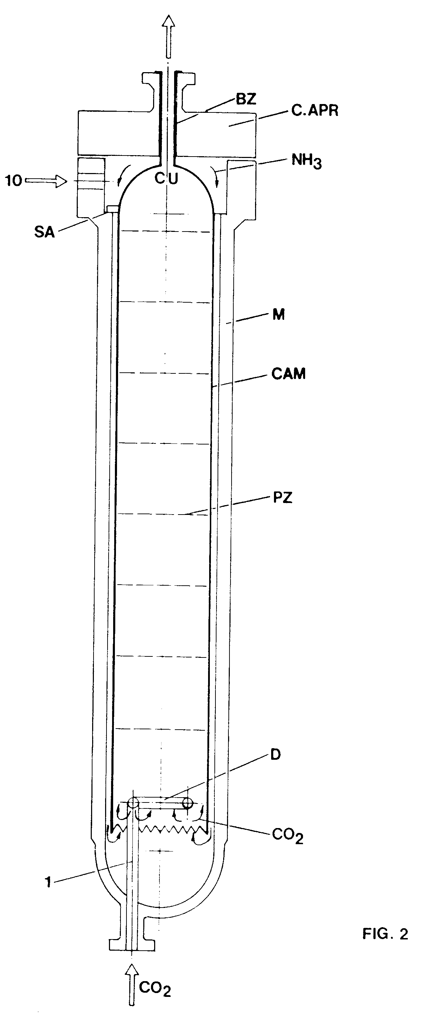

Figure 2 shows a particularly advantageous embodiment for RU reactors likely to have top openings and extractions and substitutions of the equipment inside M (CAM jacket included).

As can be seen, introduction 10 of the NH3 is now carried out above SA; the flow of NH3 therefore invests also the dome-shaped part CU which no longer needs to be in zirconium.

Only the plates (PZ) and nozzles (BZ) are still in zirconium. Welding (SA) is such as to make NH3 flow throughout the whole gap (GAP).

## Claims

1. Process for the high-yield synthesis of urea from ammonia and carbon dioxide fed in a pure state to a once-through reactor, i.e. without recycling, operating at a high pressure and temperature, characterized by the fact that the pure ammonia is fed to the reaction space after being first circulated substantially over the entire inner wall or reactor shell, the temperature of said ammonia and its circulation being such as to: a) cool said reactor shell so that it no longer requires lining with costly special material; and b) not to affect in any appreciable way the reaction.

2. Process according to claim 1, characterized by the fact that the ammonia is fed at temperatures below 150°C, preferably at about 80°C, and circulates so as to lower the temperature of the reaction shell sufficiently to avoid corrosive phenomena.

3. Process according to claim 1 or 2, in which the synthesis reaction is effected at temperatures around 190÷240°C, and more particularly at 215°C, and at a pressure from 200 to 450 bar, and more particularly at 400 bar, the ammonia is fed at 50÷150°C, and more particularly 80°C, the CO2 is fed at 120÷150°C, with the introduction of oxygen as passivating agent.

4. Reactor for carrying out the process according to the foregoing claims, comprising a shell or pressure-resistant body and lining for the latter, characterized by the fact that the lining, no longer made of costly special material but in stainless steel, is a jacket (CAM) welded to the shell (M) in the upper part (SA) and free in the lower part (EI) creating with the shell (M) an airspace (GAP) for the circulation of relatively cool ammonia.

5. Reactor according to claim 4, characterized by the fact that the NH3 feed duct (10) is just below the welding (SA) of the stainless steel jacket lining (CAM), only the dome-shaped upper part (CU) of the reactor (RU) being provided with a zirconium lining.

6. Reactor according to claim 4, characterized by the fact that the upper part (C.APR) of the reactor (RU) is removable, the NH3 feed duct (10) is placed high, above (SA) so that the NH3 flows also into the dome-shaped area (CU), only the nozzles (BZ) in contact with the upper opening part (C.APR) being lined with zirconium.

FIG. 1

FIG. 2

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| A | FR-A-2 277 618 (MITSUI TOATSU CHEMICALS)<br>* figure 1 *<br>--- | | C07C273/04<br>B01J4/00<br>B01J10/00 |
| A | US-A-3 932 504 (CHEN ET AL)<br>* figure 1 *<br>----- | | |

TECHNICAL FIELDS SEARCHED (Int. Cl.5 )

C07C
B01J

The present search report has been drawn up for all claims

| Place of search | Date of completion of the search | Examiner |
|---|---|---|
| THE HAGUE | 24 FEBRUARY 1992 | J. VAN GEYT |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)